# EUROPÄISCHE PATENTSCHRIFT

(11) **EP 1 937 707 B1**
(45) Veröffentlichungstag und Bekanntmachung des Hinweises auf die Patenterteilung: **29.12.2010**
(21) Anmeldenummer: 06807177.8
(22) Anmeldetag: 12.10.2006
(51) Int. Cl.: C07J 63/00

(54) **VERBESSERTES VERFAHREN ZUR GEWINNUNG VON BETULINSÄURE**
IMPROVED METHOD FOR THE PRODUCTION OF BETULINIC ACID
PROCEDE DE PREPARATION D'ACIDE BETULINIQUE AMELIORE

(30) Priorität: 12.10.2005 EP 05109482
(43) Veröffentlichungstag der Anmeldung: 02.07.2008
(73) Patentinhaber: Boehringer Ingelheim Pharma GmbH & Co. KG, 55216 Ingelheim (DE)
(72) Erfinder: PUDER, Carsten H., 55218 Ingelheim Am Rhein (DE); GRAEF, Herbert, 55437 Ober-Hilbersheim (DE); THUMERER, Markus J., 55118 Mainz (DE); HEITZMANN, Markus, 55218 Ingelheim Am Rhein (DE)
(74) Vertreter: Hammann, Heinz
(86) Internationale Anmeldenummer: PCT/EP2006/067309
(87) Internationale Veröffentlichungsnummer: WO 2007/042542

(56) Entgegenhaltungen:
- WO-A-03/066659
- DE-A1- 19 713 768
- BRUCKNER V ET AL: "OCCURRENCE OF BETULINIC ACID IN THE BARK OF THE PLANE TREE" JOURNAL OF THE CHEMICAL SOCIETY, CHEMICAL SOCIETY, LONDON, GB, 1948, Seiten 948-951, XP001080318 ISSN: 0368-1769 in der Anmeldung erwähnt
- DATABASE CA [Online] CHEMICAL ABSTRACTS SERVICE, COLUMBUS, OHIO, US; XIA, YUJIANG ET AL: "Method for extracting and purifying betulin and betulic acid" XP002373958 gefunden im STN Database accession no. 2006:53507 -& CN 1 634 972 A (ZHANG, MEI, PEOP. REP. CHINA) 6. Juli 2005 (2005-07-06)

## Beschreibung

### HINTERGRUND DER ERFINDUNG

Die Erfindung betrifft ein großtechnisches Verfahren zur Gewinnung von hochreiner Betulinsäure aus gemahlener Platanenrinde. Betulinsäure ist 3β-Hydroxy-lup-20(29)-en-28-säure der Formel

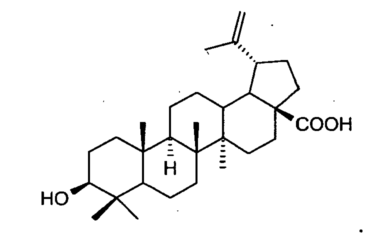

Es ist bekannt, dass Betulinsäure eine Wirkung gegen das Wachstum von Melanomzellen (z.B. Pisha et al., Nature Medicine 1, 1995, 1046 ff) sowie gegen andere Krebszellen (z.B. Sunder et al., US Patent US 6,048,847) aufweist. Des Weiteren sollen seine Derivate gegen HIV eingesetzt werden können (z.B. Evers et al., J. Med. Chem. 39, 1996, 1056 ff; Soler et al., J. Med. Chem. 39, 1996, 1069 ff oder Butler, Nat. Prod. Rep., 2005, 22, 162 ff). Demzufolge besteht ein großer Bedarf an Betulinsäure.

Neben der synthetischen Herstellung (z.B. L. Ruzicka et al., Helv. Chim. Acta 21, 1938, 1076 ff) kann Betulinsäure aus verschiedenen Pflanzen, insbesondere Bäumen gewonnen werden, so zum Beispiel aus der Rinde von *Picramnia pentandra* (z.B. Herz et al., Phytochemistry 11, 1972, 3061 ff), aus der Rinde von *Arbutus menziesii* (Robinson et al., Phytochemistry 9, 1970, 907 ff) und aus der Rinde von *Ziziphus mauritiana* (z.B. Pisha et al., Nature Medicine 1, 1995, 1046 ff).

Aus diesen Ausgangsmaterialien lässt sich die Betulinsäure nur schwer isolieren. Aussichtsreicher dagegen erscheint ihre Gewinnung aus der Rinde und/oder Borke der Platane. In der DE 197 13 768 wird ein Verfahren zur Gewinnung von Betulinsäure vorgeschlagen, wobei ein aus Platanenborken gewonnenes Pulver mit einem mittelpolaren Lösungsmittel, wie zum Beispiel Dichlormethan, Chloroform oder Diethylether extrahiert wird.

Dieses Verfahren ist jedoch für eine industrielle Gewinnung von großen Mengen an Betulinsäure nicht geeignet, da sehr große Volumina der mittelpolaren Lösungsmittel eingesetzt werden müssen, um die Betulinsäure zu extrahieren (Einsatz von 7 Litern Dichlormethan auf 150 g Platanenborken-Pulver). Des Weiteren ist die in einer Glassäule unter hydrostatischem Druck durchgeführte Extraktion für Großmengen nicht geeignet und aus Sicherheitsgründen problematisch.

Bruckner et al., J. Chem. Soc. 1948, 948-951 beschreiben ein Verfahren zur Gewinnung von Betulinsäure aus Platanenborken, wobei die gemahlenen Borken mit Methanol extrahiert werden, der gewonnene Extrakt eingeengt und das Konzentrat in Gegenwart von Kohle mehrfach aus Methanol umkristallisiert wird. Die so gewonnene Betulinsäure weist jedoch noch eine Vielzahl von Verunreinigungen auf.

WO 03/066659 offenbart ein Verfahren zur Gewinnung von Betulinsäure aus einem methanolischen Extrakt von gemahlener Platanenrinde. Die Ausbeute beträgt in Rohform 2,3% bezogen auf die eingesetzte Platanenrinde, weist jedoch nicht die gewünschte Reinheit auf, obwohl ein zusätzlicher Reinigungsschritt, welcher das Ausrühren des Extraktes mit n-Hexan beinhaltet, durchgeführt wird.

Daher ist es Aufgabe der vorliegenden Erfindung ein verbessertes Verfahren zur Gewinnung von Betulinsäure aus Platanenrinde, das auch für den großtechnischen Einsatz geeignet ist, bereitzustellen.

### DETAILLIERTE BESCHREIBUNG DER ERFINDUNG

Überraschenderweise wurde nun gefunden, dass sich bei dem erfindungsgemäßen Verfahren der Umsatz deutlich erhöhen lässt. Weiterhin lässt sich durch Vermeidung des Einsatzes von Chromatographiematerial die Abfallmenge klein halten.

Dementsprechend liegt der vorliegenden Erfindung ein verbessertes Verfahren zur Gewinnung von hochreiner Betulinsäure durch Extraktion von gemahlener Platanenrinde und/oder Platanenborke zu Grunde, das dadurch gekennzeichnet ist, dass die Extraktion mit einem Lösungsmittelgemisch bestehend aus Toluol
einem oder mehreren C₁₋₆-Alkoholen
durchgeführt wird.

Besonders bevorzugt wird eine Lösungsmittelgemisch aus Toluol und
- Methanol, Ethanol, *n*-Propanol, 2-Propanol, *n*-Butanol *t*-Butanol, *n*-Pentanol, (+-)-2-Pentanol oder 3-Pentanol, oder

Ein bevorzugtes Lösungsmittelgemisch besteht aus Toluol und zwei Alkoholen ausgewählt aus der Gruppe bestehend aus Methanol, Ethanol, *n*-Propanol, 2-Propanol, *n-*Butanol, *t*-Butanol, *n*-Pentanol, (+-)-2-Pentanol oder 3-Pentanol. Besonders bevorzugt besteht das Lösungsmittelgemisch aus Toluol, Methanol und einem zweiten Alkohol (Ethanol, *n*-Propanol, 2-Propanol, *n*-Butanol, *t*-Butanol, *n*-Pentanol, (+-)-2-Pentanol oder 3-Pentanol), insbesondere bevorzugt aus Toluol, Methanol und (+-)-2-Pentanol. Durch das Verfahren der Erfindung lässt sich das eingesetzte Lösungsmittelgemisch sehr gut zurückgewinnen, weshalb der Lösungsmittelverlust beim Recyclingprozess gering ist, als geeignet hierfür hat sich Toluol in einem Verhältnis zu den oben genannten Alkoholen zwischen 80:20 und 95:5 erwiesen.

Besonders bevorzugt ist das obige Verfahren, worin man die Platanenrinde vor der Extraktion mit Wasser anfeuchtet. Dies kann zum einen die Verarbeitbarkeit der Platanenrinde verbessern und zum anderen die Belastung der Umwelt durch Stäube vermindern.

Das voran beschriebene Verfahren kann kontinuierlich oder als Batch-Verfahren durchgeführt werden, bevorzugt ist das obige Verfahren worin die Extraktion kontinuierlich erfolgt.

Bevorzugt erfolgt das Verfahren kontinuierlich mit einer Flussgeschwindigkeit des Lösungsmittelgemisches von 0.2 m³/h bis 6 m³/h innerhalb einer Dauer von 2 h bis 48 h, bevorzugt 5 h bis 35 h, insbesondere 15 h bis 25 h. Dabei können Batterie-Extraktionsanlagen variabler Größe und Bauart eingesetzt werden. Die durchschnittliche Tagesmengen an Platanenrinden-Pulver für die Extraktion kann zwischen 500 kg und 8 Tonnen liegen.

Insbesondere bevorzugt wird das obige Verfahren worin die Extraktion bei einer Temperatur von 20 bis 110°C, bevorzugt 40 bis 90°C, besonders bevorzugt 50 bis 70°C erfolgt.

Zur Gewinnung der Betulinsäure aus dem rohen Extrakt werden dem Verfahren noch weitere Schritte angeschlossen. Gemäß dem Verfahren der Erfindung werden nacheinander folgende Schritte zum Isolieren der Betulinsäure durchgeführt.
a) Extraktion von Platanenrinde in einem Lösungsmittelgemisch wie oben definiert;
b) Abziehen des Lösungsmittelsgemisches aus dem Extrakt;
c) Lösen des Rückstandes in einem geeigneten Lösungsmittel;
d) Abscheiden von Betulinsäure aus dem Filtrat.

Als geeignete Lösungsmittel in Schritt c) kommen Mischungen aus C₁₋₄-Chloralkanen oder Estern der Essigsäure und C₁₋₄-Alkoholen in Frage. Bevorzugte Chloralkane sind Dichlormethan und Chloroform, bevorzugte Ester der Essigsäure sind Essigsäuremethylester, Essigsäureethylester, Essigsäure-2-propylester und bevorzugte Alkohole sind Methanol, Ethanol und 2-Propanol. Besonders bevorzugt ist eine Mischung aus Dichlormethan und Methanol in Verhältnissen von 5:1 bis 1:2, bevorzugt 3:1 bis 1:1, besonders bevorzugt 2:1 bis 1.5:1. Dabei wird eine geeignete Menge an Lösungsmittel oder Lösungsmittelgemisch bezogen auf den festen Rückstand eingesetzt. Bevorzugt wird ein Verhältnis von 5 bis 30 Liter, besonders bevorzugt 16 bis 20 Liter pro Kilogramm Feststoff. Das Abscheiden der Betulinsäure erfolgt dabei bevorzugt durch langsames Abkühlen des Filtrats auf -15 bis 10°C, bevorzugt -10 bis 10°C, besonders bevorzugt 0 bis 10°C innerhalb von einem Zeitraum von 5 bis 50 h, bevorzugt 5 bis 20 h, besonders bevorzugt 10 bis 15 h. Anschließend kann die gereinigte Betulinsäure als Feststoff durch im Stand der Technik bekannte Verfahren (Nutsche, Zentrifuge, Dekanter, Druckfilter, Filtertrockner etc.) von der Mutterlauge abgetrennt und getrocknet werden. Die so gereinigte Betulinsäure kann dabei immer noch bis zu 10% Lösungsmittel enthalten.

Ein weiterer Zwischenschritt kann die Reinheit der Betulinsäure deutlich erhöhen und die weitere Bearbeitung vereinfachen. Bevorzugt ist das das obige Verfahren worin man zwischen Schritt b) und c) folgende Schritte durchführt:
b1) Suspendieren und Lösen des Rückstandes in Propanol, Butanol oder einem Gemisch davon;
b2) Versetzten mit einer Filter- oder Aufsaugmasse
b3) Filtrieren der Suspension
b4) Abscheiden der rohen Betulinsäure aus dem filtrierten Extrakt.

Die Schritte b1) bis b4) können gegebenenfalls einfach oder mehrfach durchgeführt werden. Bevorzugt erfolgt das Suspendieren in Schritt b1) in Propanol, insbesondere 2-Propanol. Dabei kann ein Volumen des Lösungsmittels von 10 bis 50 l, bevorzugt 15 bis 25 l, besonders bevorzugt 17 bis 19 l pro Kilogramm des lösungsmittelfreien Extraktes, eingesetzt werden. Bevorzugt wird das Filtrat aus Schritt b3) vor dem Abscheiden in Schritt b4) eingeengt, besonders bevorzugt auf eine Verdünnung von 1:12 bis 1:36 zum entstehenden bzw. erwarteten Feststoff. Das Abscheiden erfolgt dabei bevorzugt durch langsames Abkühlen des Filtrats auf -15 bis 15°C, bevorzugt -5 bis 15°C, besonders bevorzugt 5 bis 15°C innerhalb von einem Zeitraum von 5 bis 50 h, bevorzugt 5 bis 20 h, besonders bevorzugt 10 bis 15 h. Anschließend kann die rohe Betulinsäure als Feststoff durch im Stand der Technik bekannte Verfahren (Nutsche, Zentrifuge, Dekanter, Druckfilter, Filtertrockner etc.) von der Mutterlauge abgetrennt und getrocknet werden. Die so erhaltene rohe Betulinsäure kann dabei immer noch bis zu 15% Lösungsmittel enthalten.

Bevorzugt besteht die die oben beschriebene Filter- oder Aufsaugmasse aus Aktivkohle und/oder Kieselgur.

Dabei können variable Mengen der Filter- oder Aufsaugmasse eingesetzt werden. Sinnvolle Mengen zum Einsatz als Filter- oder Aufsaugmasse in einem Reinigungsschritt sind dem Fachmann geläufig.

### BEGRIFFE UND DEFINITIONEN

Der Begriff "Betulinsäure" wie er vor- und nachstehend verwendet wird, umfasst sowohl Betulinsäure als solche sowie ihre Hydrate und Solvate, vorzugsweise Betulinsäure, welche mit 0.5 bis 2 Äquivalenten eines Lösungsmittels solvatisiert ist.

Im Rahmen der Erfindung sind alle Arten der Gattung Platanus geeignet. Als besonders geeignete Platanen haben sich die Amerikanische oder Westliche Platane (*Platanus occidentalis*), die Morgenländische oder Orientalische Platane (*Platanus orientalis*), die Kerrs Platane (*Platanus kerrii*), die Mexikanische Platane (*Platanus mexicana*), die Kalifornische Platane (*Platanus racemosa*) und die Arizona Platane (*Platanus wrightii*) herausgestellt. Aber auch Platanen-Unterarten sowie aus Arten bzw. Unterarten abgeleitete Hybride wie z.B. die Ahornblättrige, Gemeine oder Bastard-Platane (*Platanus x hispanica lacerifolia*) (Hybrid aus *P. occidentalis* und *P. orientalis*) sind als Ausgangsmaterial zur Gewinnung von Betulinsäure geeignet.

Unter dem Begriff "C₁₋₄-Alkohol" werden verzweigte und unverzweigte Alkohole mit 1 bis 4 Kohlenstoffatomen und einer oder zwei Hydroxygruppen verstanden. Beispielsweise werden hierfür genannt: Methanol, Ethanol, *n*-Propanol, 2-Propanol, *n*-Butanol, *iso-*Butanol, *sec*-Butanol oder *tert*-Butanol. Gegebenenfalls werden für vorstehend genannte Moleküle auch die Abkürzungen MeOH, EtOH, *n*-PrOH, 2-PrOH, *n*-BuOH, *i*-BuOH, *t-*BuOH, etc. verwendet. Sofern nicht anders beschrieben, umfassen die Definitionen Propanol und Butanol alle denkbaren isomeren Formen der jeweiligen Reste. So umfasst beispielsweise Propanol *n*-Propanol und 2-Propanol, Butanol umfasst *iso*-Butanol, *sec-*Butanol und *tert*-Butanol etc.

Unter dem Begriff "C₁₋₄-Chloralkane" werden verzweigte und unverzweigte Alkane mit 1 bis 4 Kohlenstoffatomen verstanden, die mit einem oder mehreren Chloratomen substituiert sind. Beispielsweise werden hierfür Dichlormethan und Chloroform genannt.

### BEISPIEL

### 1A HERSTELLUNG VON PLATANENRINDEN-EXTRAKT

Zur Extraktion von Platanenrinde-Pulver im großtechnischen Maßstab wird eine aus drei Extraktoren, einer Reindestillat-Vorlage und einem Verdampfer bestehende Batterie-Extraktionsanlage eingesetzt. Zum vollständigen Beschicken der Anlage werden pro Extraktor 650 kg Platanenrinde-Pulver mit 260 l Wasser in einem Mischer vermischt und über ein Förderband in den jeweiligen Extraktor eingetragen. Jeder einzelne Extraktor wird anschließend noch mit 2.5 m³ warmen Toluol/(+-)-2-Pentanol/Methanol-Gemisch (88:7:5) aufgefüllt. Mit einer Flussrate von 2 m³/h wird von der Reindestillat-Vorlage ausgehend, auf 60 °C erwärmtes Toluol/(+-)-2-Pentanol/Methanol-Gemisch (88:7:5) durch die drei in Reihe geschalteten Extraktoren gepumpt. Pro Stunde werden aus dem System ebenfalls 2 m³ Extrakt in den Verdampfer eingespeist und dort durch Abdestillieren des Lösungsmittelgemisches aufkonzentriert.
Das anfallende Destillat wird mittels eines Phasenabscheiders von dem mit abdestillierten Wasser befreit und die organische Phase anschließend gaschromatographisch analysiert. Falls notwendig, stellt man die Zusammensetzung der organischen Phase durch Zugabe von frischem Methanol bzw. (+-)-2-Pentanol wieder auf 88:7:5 für Toluol/(+-)-2-Pentanol/Methanol ein. Das auf diese Weise recycelte Toluol/(+-)-2-Pentanol/Methanol-Gemisch wird in die Reindestillat-Vorlage eingespeist und auf 60 °C temperiert.
Nach 20 h wird der Extraktionsvorgang abgebrochen. Die in den Extraktoren enthaltene Restmenge des Extraktes wird abgepumpt und ebenfalls im Verdampfer eingeengt. Die komplett angefallene Extraktmenge wird auf ein Volumen von 1300 l aufkonzentriert. Der mittels Rückstandsbestimmung ermittelte Gehalt des Gesamtextraktes liegt bei 61 g/l.

### 1 B GEWINNUNG VON BETULINSÄURE ROH

1300 l des nach Beispiel 1A erhaltenen Extraktes werden vom Verdampfer in eine Destillationsapparatur umgepumpt, dann bis zur Trockne eingedampft und der Rückstand in 560 l 2-Propanol suspendiert. Es wird erneut bis zur Trockne eingedampft und anschließend der Destillationsrückstand mit 1430 l 2-Propanol unter Rückfluss unter Normaldruck 30 min erhitzt. Die Suspension wird mit 11 kg Kieselgur und 24 kg Aktivkohle versetzt und über einen Druckfilter in einen Kristaller heiß filtriert und die Destillationsapparatur und der Druckfilter mit 20 12-Propanol, gespült. Von der 2-propanolischen Lösung werden 240 l 2-Propanol abdestilliert und anschließend wird der Kristallerinhalt über Nacht auf 10 °C abgekühlt. Die dabei entstandene Suspension wird über eine Filternutsche abgesaugt, der Feststoff mit 48 l 2-Propanol gewaschen und danach bei 55 °C im Vakuumtrockenschrank getrocknet. Das 2-Propanol kann durch Destillation zurück gewonnen werden.
Man erhält 43.4 kg Betulinsäure roh. Smp. 309-310 °C; Betulinsäuregehalt (HPLC-UV): 83.9%; 2-Propanolgehalt (¹H NMR):10.5%.

### 1C GEWINNUNG VON BETULINSÄURE REIN

43.4 kg Betulinsäure roh erhalten nach Beispiel 1 B werden in einer Destillationsapparatur mit 930 l Dichlormethan und 325 l Methanol unter Rückfluss erhitzt und gelöst. Man lässt auf 30 °C abkühlen und gibt eine Suspension von 22 kg Aktivkohle und 11 kg Kieselgur in 65 l Methanol hinzu. Der Apparateinhalt wird erneut bis zum Sieden erhitzt, heiß über einen Druckfilter in einen Kristaller filtriert und die Destillationsapparatur und der Druckfilter mit 50 l Dichlormethan gespült. Von dem Filtrat werden unter Normaldruck 630 l abdestilliert und der Apparateinhalt anschließend über Nacht auf 5 °C abgekühlt. Der dabei ausgefallene Feststoff wird über eine Zentrifuge abgetrennt, mit 44 l Methanol bei 5 °C gedüst und anschließend bei 50 °C im Vakuumtrockenschrank getrocknet. Man erhält 28.9 kg Betulinsäure rein. Smp. 308-310 °C; Betulinsäuregehalt (HPLC-UV): 93.8%; Dichlormethangehalt (¹H NMR): 2.1%; Methanolgehalt (¹H NMR): 2.3%.

## Patentansprüche

1. Verfahren zur Gewinnung von hochreiner Betulinsäure durch Extraktion von gemahlener Platanenrinde und/oder Platanenborke, **dadurch gekennzeichnet, dass** man nacheinander folgende Schritte zum isolieren der Betulinsäure durchführt:
a) Extraktion von Platanenrinde in einem Lösungsmittelgemisch bestehend aus Toluol und einem oder mehreren C₁₋₆-Alkoholen in einem Verhältnis von Toluol zu den genannten Alkoholen zwischen 80:20 und 95:5:
b) Abziehen des Lösungsmittelgemisches aus dem Extrakt;
c) Lösen des Rückstandes in einem geeigneten Lösungsmittel;
d) Abscheiden von Betulinsäure aus dem Filtrat.

2. Verfahren nach Anspruch 1 **dadurch gekennzeichnet, dass** man die Platanenrinde vor der Extraktion mit Wasser anfeuchtet.

3. Verfahren nach einem der Ansprüche 1 oder 2 **dadurch gekennzeichnet, dass** die Extraktion kontinuierlich erfolgt.

4. Verfahren nach einem der Ansprüche 1 bis 3 **dadurch gekennzeichnet, dass** die Extraktion bei einer Temperatur von 20 bis 110°C erfolgt.

5. Verfahren nach einem der Ansprüche 1 bis 4 **dadurch gekennzeichnet, dass** man zwischen Schritt b) und c) folgende Schritte durchführt:
b1) Suspendieren und Lösen des Rückstandes in Propanol, Butanol oder einem Gemisch davon;
b2) Versetzten mit einer Filter- oder Aufsaugmasse
b3) Filtrieren der Suspension
b4) Abscheiden der rohen Betulinsäure aus dem filtrierten Extrakt.

6. Verfahren nach Anspruch 5 **dadurch gekennzeichnet, dass** die Filter- oder Aufsaugmasse aus Aktivkohle und/oder Kieselgur besteht.

## Claims

1. Process for obtaining highly pure betulinic acid by extraction of ground plane bark and/or cortex, **characterised in that** the following steps are carried out in succession in order to isolate the betulinic acid:
a) extraction of plane bark in a solvent mixture consisting of toluene and one or more C₁₋₆-alcohols in a ratio of toluene to the above-mentioned alcohols of between 80:20 and 95:5;
b) removal of the solvent mixture from the extract;
c) dissolving the residue in a suitable solvent;
d) precipitating betulinic acid from the filtrate.

2. Process according to claim 1, **characterised in that** the plane bark is moistened with water before the extraction.

3. Process according to one of claims 1 or 2, **characterised in that** the extraction is carried out continuously.

4. Process according to one of claims 1 to 3, **characterised in that** the extraction is carried out at a temperature of 20 to 110°C.

5. Process according to one of claims 1 to 4, **characterised in that** between step b) and c) the following steps are carried out:
b1) suspending and dissolving the residue in propanol, butanol or a mixture thereof;
b2) mixing with a filtering or suction composition
b3) filtering the suspension
b4) precipitating the crude betulinic acid from the filtered extract.

6. Process according to claim 5, **characterised in that** the filtering or suction composition consists of activated charcoal and/or kieselguhr.

## Revendications

1. Procédé d'obtention d'acide bétulinique de grande pureté par extraction d'écorce de platane broyée, **caractérisé en ce que** l'on réalise successivement les étapes suivantes pour isoler l'acide bétulinique :
a) Extraction de l'écorce de platane dans un mélange de solvants consistant en le toluène et en un ou plusieurs alcools en C₁₋₆ en un rapport du toluène aux alcools cités allant de 80:20 à 95:5 ;
b) Élimination du mélange de solvant de l'extrait ;
c) Dissolution du résidu dans un solvant approprié ;
d) Précipitation de l'acide bétulinique à partir du filtrat.

2. Procédé selon la revendication 1, **caractérisé en ce que** l'on humidifie l'écorce de platane avec l'eau avant l'extraction.

3. Procédé selon la revendication 1 ou 2, **caractérisé en ce que** l'extraction est réalisée de manière continue.

4. Procédé selon l'une des revendications 1 à 3, **caractérisé en ce que** l'extraction est réalisée à une température allant de 20 à 110°C.

5. Procédé selon l'une des revendications 1 à 4, **caractérisé en ce que** l'on réalise entre les étapes b) et c), les étapes suivantes :
b1) Mise en suspension et dissolution du résidu dans le propanol, le butanol ou un mélange de ceux-ci ;
b2) Addition d'une masse de filtration ou d'absorption ;
b3) Filtration de la suspension ;
b4) Précipitation de l'acide bétulinique brut depuis l'extrait filtré.

6. Procédé selon la revendication 5, **caractérisé en ce que** la masse de filtration ou d'absorption consiste en du charbon activé et/ou la terre d'infusoires.
